# EUROPEAN PATENT APPLICATION

(11) **EP 3 747 476 A1**
(43) Date of publication of application: **09.12.2020**
(21) Application number: 19747012.3
(22) Date of filing: 28.01.2019
(51) Int. Cl.: A61L 27/36, A61L 27/46, A61L 27/54, A61L 27/20

(54) **COMPOSITION FOR BONE GRAFTING, COMPRISING NUCLEIC ACIDS, BONE GRAFT MATERIAL AND CATIONIC POLYMER, AND BONE GRAFT KIT FOR MANUFACTURING SAME**

(30) Priority: 30.01.2018 KR 20180011586
(71) Applicant: Pharmaresearch Products Co., Ltd., Gangneung-si, Gangwon-do 25452 (KR)
(72) Inventor: KIM, Ik Soo, Seongnam-si, Gyeonggi-do 13568 (KR); SHIN, Chul Ho, Gwangju-si, Gyeonggi-do 12767 (KR); PARK, Min Hyeong, Changwon-si, Gyeongsangnam-do 51747 (KR); LEE, Su Yeon, Seongnam-si, Gyeonggi-do 13585 (KR); KIM, Tae Gyun, Seoul 06213 (KR); LEE, Sung Oh, Seongnam-si, Gyeonggi-do 13508 (KR); SEO, Han Sol, Seongnam-si Gyeonggi-do 13186 (KR); KONG, Byoung Hwan, Hwaseong-si, Gyeonggi-do 18486 (KR); LEE, Jeong Kuk, Yongin-si, Gyeonggi-do 16931 (KR)
(74) Representative: Beckmann, Claus
(86) International application number: PCT/KR2019/001176
(87) International publication number: WO 2019/151736

(57) **Abstract**

The present invention relates to a composition for bone grafting, comprising nucleic acids, a bone graft material, and a cationic polymer, and a bone graft kit for manufacturing the same. The composition for bone grafting, of the present invention, has been confirmed to promote the formation of a cushioning force that can respond to physiological stress and the formation of new bones at grafted sites, and has been confirmed to improve bone grafting convenience, and thus is expected to be effectively usable in the treatment of bone diseases.

## Description

### Technical Field

The present invention relates to a bone grafting composition comprising a nucleic acid, a bone graft material, and a cationic polymer and a bone grafting kit for manufacturing the same.

### Background Art

Skeletal diseases have recently occurred at increasing frequencies. In addition, patients suffering from bone tissue-related diseases have become more and more abundant with the development of sports and the increase of a desire for improving the quality of life.

To repair bone deformities caused by innate or acquired bone disease or bone defects caused by external stimuli or damage, grafts replaceable for bones are introduced into the patients. In this regard, the available bone grafts may be divided into autogeneic, allogeneic, xenogeneic, and synthetic bones according to the origins thereof. Of the bone grafts, autogeneic bones are the most ideal because of the excellent osteoinductivity and bone morphogenesis thereof. However, autogeneic bones require additional surgeries for the collection thereof, which may result in the accompaniment of complications and an increased recovery time. Other problems that are encountered along with the need of autogeneic bones include the impossibility to obtain a sufficient amount thereof and the increase in time taken to conduct the subsidiary surgery for bone collection.

In order to overcome such problems, development and studies of various bone grafts for use as alternatives to autogeneic bones continue to be undertaken. Recently, extensive research has been conducted into synthetic bone grafts. In addition, the application of existing synthetic bones, osteoconductive materials, and osteoinductive materials in combination is also actively studied to promote bone morphogenesis.

Furthermore, research is directed to bone scaffolds that support damaged or defective bone structures and induce the formation of new bone at defective sites from neighboring tissues. For use in inducing such bone morphogenesis, a bone scaffold should ideally meet the conditions of: being excellently bioactive, being free of infection and inflammatory reactions, ease of supporting and filling bone defect sites, having an in vivo degradable 3D structure able to function as a channel for osteoconductive materials, and having a cushioning force to respond to a biological stress applied to a grafted site (Guarino V., et al., 2007) . However, there is contradiction that the cushioning force decreases when in vivo activity and absorptivity are improved. It is thus important to develop a scaffold that avoids and reduces the contradiction.

Leading to the present invention, thorough and intensive research into bone regeneration with nucleic acid and cationic polymer mixtures, conducted by the present inventors, culminated in the finding that bone graft materials are held by bonds between nucleic acids and cationic polymers to form aggregates which are also observed to have the ability to prevent the bone graft materials from being scattered and retain constant shapes, thereby providing a suitable environment in which the convenience of grafting can be improved and bone morphogenesis can be induced through the retainability to preserve the shape of the aggregates and the cushioning force to absorb impacts.

With respect to related arts, reference may be made to Korean Patent No. 1488716, which discloses a kit comprising PDRN (nucleic acid) and chitosan (cationic polymer) for regenerating alveolar bones, but does not mention the disclosure of the present invention on the aggregation of bone grafts by bonds between nucleic acids and cationic polymers and the resultant effect of improving the convenience of bone grafting. In addition, Korean Patent Number 1161784 discloses the aggregating agent for bone powder, which comprises a nucleic acid or chitosan, but does not mention the disclosure of the present invention on the effect of aggregating bone grafts by bonds between nucleic acids and cationic polymers. Europe Patent Number 2745849 A1 discloses a composition comprising PDRN and chitosan and its effect of stimulating cell proliferation necessary for regenerative medicine. Korean Patent Number 1710615 discloses a filling solution comprising a nucleic acid and chitosan and its effect of improving engrafting rates in the dermal layer. However, the disclosure of the present invention on the role of a nucleic acid and chitosan in forming an aggregate of a bone graft and the effect thereof are stated in neither of the cited documents.

Turning to non-patent documents, Kim, S.K., et al. disclose a bone regeneration effect of PDRN, but does not state the disclosure of the present invention on the aggregation of a bone graft by a bond between a nucleic acid and a cationic polymer and the improved convenience of bone grafting. In another non-patent document, Martin R.V., et al. states the function of chitosan as a scaffold and to regenerate bone in the tissue regeneration field, but not the disclosure of the present invention on the aggregation of a bone graft by a bond between a nucleic acid and a cationic polymer.

### Detailed Description of the Invention

### Technical Problem

A purpose of the present invention is to provide a composition comprising a nucleic acid, a bone graft, and a cationic polymer for bone grafting, and a bone graft kit for manufacturing the same.

### Technical Solution

The present invention is directed to a bone grafting composition, comprising: a) a nucleic acid; b) a bone graft; and c) a cationic polymer, wherein a) the nucleic acid and c) the cationic polymer form a bond which holds b) the bone graft to form an aggregate.

The bone grafting composition may be prepared by a process comprising: a first step of mixing a) the nucleic acid with b) the bone graft to give a nucleic acid-bone graft mixture and a second step of adding c) the cationic polymer to the nucleic acid-bone graft mixture to hold the bone graft to form an aggregate.

The bone grafting composition may be prepared by a process comprising: a first step of mixing c) the cationic polymer with b) the bone graft to give a cationic polymer-bone graft mixture; and a second step of adding a) the nucleic acid to the cationic polymer-bone graft mixture to hold the bone graft to form an aggregate.

The bond may be an ionic bond.

In the bone grafting composition, the nucleic acid may be mixed at a weight ratio of 1-20:1 with the cationic polymer.

The nucleic acid may be contained in an amount of 0.001-2 % by weight, based on the total weight of the bone grafting composition.

The nucleic acid may be a deoxyribonucleic acid (DNA), a ribonucleic acid (RNA), or a mixture thereof.

The nucleic acid may have a molecular weight of 1-100,000 kDa.

The cationic polymer may be contained in an amount of 0.001-2 % by weight, based on the total weight of the bone grafting composition.

The cationic polymer may be at least one selected from the group consisting of chitosan, polyethylene amine, poly-L-lysine, and polyallylamine, and particularly may be chitosan.

The bone graft may be an autogeneic demineralized bone matrix, an allogeneic demineralized bone matrix, a xenogeneic demineralized bone matrix, a synthetic bone graft, an autologous bone homogenate, or a mixture thereof.

The bone grafting composition may further comprise a bone morphogenesis activator.

The bone morphogenesis activator may be a growth factor, a hemostatic, or a mixture thereof.

The growth factor may be at least one selected from the group consisting of bone morphogenetic protein (BMP), bone sialoprotein, transforming growth factor (TGF), platelet-derived growth factor (PDGF), platelet rich plasma (PRP), fibroblast growth factor (FGF), dentin sialoprotein, polydeoxyribonucleotide, heparin-binding EGF-like growth factor (HB-EGF), cadherin EGF LAG seven-pass G-type receptor 3, and osteoblast specific cadherin (OB-cadherin).

The hemostatic may be at least one selected from the group consisting of thrombin, thromboplastin, fibrinogen, casein-kinase II, tissue factor, epinephrine, gelatin, vitamin K, calcium chloride, aluminum chloride-hexahydrate, and aluminum sulfate. Also, the present invention concerns a bone grafting kit for preparing the bone grafting composition, the bone grafting kit comprising: a-1) a nucleic acid solution; b) a bone graft; and c-1) a cationic polymer solution.

The bone grafting kit may comprise a bone morphogenesis activator.

Below, a detailed description will be given of the present invention.

The present invention relates to a bone grafting composition comprising: a) a nucleic acid; b) a bone graft; and c) a cationic polymer; wherein a bond between a) the nucleic acid and c) the cationic polymer holds b) the bone graft to form an aggregate.

In the bone grafting composition, b) the bone graft is held by a bond appearing between a) the nucleic acid and c) the cationic polymer, whereby an aggregate is formed. In preparing the bone grafting composition, the mixing order of a) the nucleic acid, b) the bone graft, and c) the cationic polymer is important.

The bone grafting composition may be prepared by a process comprising: a first step of mixing a) a nucleic acid with b) a bone graft to give a nucleic acid-bone graft mixture; and a second step of adding c) a cationic polymer to the nucleic acid-bone graft mixture to hold the bone graft to form an aggregate or by a process comprising: a first step of mixing c) a cationic polymer with b) a bone graft to give a cationic polymer-bone graft mixture; and a second step of adding a) a nucleic acid to the cationic polymer-bone graft to hold the bone graft to form an aggregate.

In a preparation process for the bond grafting composition, when a) a nucleic acid is mixed with c) a cationic polymer and then added with b) a bone graft, a bond is first formed between the nucleic acid and the cationic polymer to form an aggregate composed of only the nucleic acid and the cationic polymer, without holding the bond graft. Thus, this approach is unsuitable for the preparation of the bone grafting composition of the present invention.

As used herein, the term "holding" refers to a state in which particles of b) the bone graft are aggregated or are not dispersed as they are supported or fixed during the formation of a bond between a) the nucleic acid and c) the cationic polymer or refers to the incorporation or inclusion of particles of b) the bone graft in a complex resulting from the bond between a) the nucleic acid and c) the cationic polymer so as to form an aggregate.

The holding state of the bone graft can be maintained even when an external impact or environmental change is applied thereto.

The bond may be an ionic bond.

The ionic bond is a bond involving the electrostatic attraction between the anionic nucleic acid and the cationic polymer, forming the bone grafting composition into an aggregate in a stable state.

In the bone grafting composition, the nucleic acid and the cationic polymer may be mixed at a weight ratio of 1-20:1 and particularly at a weight ratio of 2-10:1. When departing from the weight ratio range, a weight ratio between the nucleic acid and the cationic polymer is not desirable because the bone grafting composition of the present invention does not aggregate at such a weight ratio.

The nucleic acid may be contained in an amount of 0.001-2 % by weight, based on the total weight of the bone grafting composition, particularly in an amount of 0.01-2 % by weight, more particularly in an amount of 0.1-2 % by weight, and most particularly in an amount of 1-2 % by weight. When the nucleic acid is contained in an amount less than 0.001 % by weight, the bone graft may not aggregate. When used in an amount exceeding 2 % by weigh, the nucleic acid may be not properly dissolved during the preparation of the composition.

In the present invention, the nucleic acid, which is used to form a bond with the cationic polymer to hold the bone graft, thereby forming an aggregate, is a polymeric material in which nucleotides, each consisting of a base, a sugar, and a phosphate, are polymerized into a long-chain molecule via phosphodiester linkages. The nucleic acid may be particularly a deoxyribonucleic acid (DNA), a ribonucleic acid (RNA), or a mixture thereof and more particularly a deoxyribonucleic acid.

The deoxyribonucleic acid may be a deoxyribonucleic acid prepared by extraction from an animal or a plant, particularly a deoxyribonucleic acid prepared by extraction from a fish, and more particularly an oligonucleotide, a polynucleotide, a polydeoxyribonucleotide, or a mixture thereof prepared by extraction from sperm or testis of a fish.

The fish may belong to the family Salmonidae and may be particularly trout or salmon, and most particularly salmon.

The nucleic acid may have a molecular weight of 1-100,000 kDa, particularly 10-10,000 kDa, and most particularly 50-3,500 kDa. When the molecular weight of the nucleic acid is below 1 kDa, the bone graft may be not easy to aggregate. A nucleic acid with a molecular weight greater than 100,000 kDa may be difficult to dissolve.

As for the cationic polymer, its amount may be 0.001-2 % by weight, based on the total weight of the bone grafting composition, particularly 0.01-2 % by weight, more particularly 0.1-2 % by weight, and most particularly 1-2 % by weight. When the cationic polymer is contained in an amount less than 0.001 % by weight, it may be difficult to aggregate the bone graft. When used in an amount greater than 2 % by weight, the cationic polymer may be difficult to dissolve in the preparation process for the composition.

Designed to form an ionic bond with the nucleic acid to hold the bone graft so as to afford an aggregate, the cationic polymer of the present invention is biocompatible and may be at least one selected from the group consisting of chitosan, polyethylene amine, poly-L-lysine, and polyallylamine, and particularly chitosan, but without limitations thereto.

The chitosan may have a molecular weight of 3-1,000 kDa, but is not limited thereto. When the chitosan has a molecular weight less than 3 kDa, the bone grafting composition is low in cohesiveness and may exhibit a poor function as a bone scaffold at a grafted site. A chitosan with a molecular weight greater than 1,000 kDa may be difficult to dissolve.

In the bone grafting composition, the nucleic acid and the cationic polymer are mixed at a weight ratio of 1-20:1. In this regard, the nucleic acid and the cationic polymer are used particularly at a content of 0.001-2 % by weight and at a content of 0.001-2 % by weight, respectively, based on the total weight of the bone grafting composition.

The bone graft may be an autogeneic demineralized bone matrix, an allogeneic demineralized bone matrix, a xenogeneic demineralized bone matrix, a synthetic bone graft, an autogeneic bone homogenate, or a combination thereof, but is not limited thereto.

The bone graft may be in a form of powders, particles, debris, blocks, pastes, or gels, particularly in a form of powders, particles, and debris, and most particularly in a form of powders.

The bone grafting composition comprises a) a nucleic acid, b) a bone graft, and c) a cationic polymer, wherein b) a bone graft is held by a bond between a) the nucleic acid and c) the cationic polymer, thereby forming an aggregate. Particularly, the bone graft is entirely immersed in the solution of the bone grafting composition so that most of the bone graft particles are held by the bond between the nucleic acid and the cationic polymer to incite aggregation. Thus, the bone graft may be mixed in an amount less than 100 % by volume, based on the total volume of the solution of the nucleic acid and the solution of the cationic polymer in the bone grafting composition, particularly in an amount of 90% or less. When the volume of the bone graft exceeds 100 % of the total volume of the nucleic acid solution and the cationic polymer solution, the nucleic acid and cationic polymer in the solution may fail to hold all the bone graft particles, making poor aggregation.

The bone grafting composition may further include a bone morphogenesis activator.

The bone morphogenesis activator may be impregnated into the bone grafting composition by immersing the bond grafting composition in a solution of the bone morphogenesis activator.

The bone morphogenesis activator may be a growth factor, a hemostatic, or a combination thereof.

The growth factor is to promote bone regeneration activity and may be at least one selected from the group consisting of bone morphogenetic protein (BMP), bone sialoprotein, transforming growth factor (TGF), platelet-derived growth factor (PDGF), platelet rich plasma (PRP), fibroblast growth factor (FGF), dentin sialoprotein, a polydeoxyribonucleotide, heparin-binding EGF-like growth factor (HB-EGF), cadherin EGF LAG seven-pass G-type receptor 3, and osteoblast specific cadherin (OB-cadherin), but without limitations thereto.

Functioning to promote wound healing, the hemostatic may be at least one selected from the group consisting of thrombin, thromboplastin, fibrinogen, casein-kinase II, tissue factor, epinephrine, gelatin, vitamin K, calcium chloride, aluminum chloride-hexahydrate, and aluminum sulfate, but is not limited thereto.

The bone grafting composition may further comprise a hydrophilic polymer. The hydrophilic polymer may be at least one selected from the group consisting of carboxymethylcellulose, alginic acid, hyaluronic acid, collagen, and a combination thereof.

The hydrophilic polymer may supplement physical properties of the bone grafting composition and augment the regeneration effect at a grafted site.

Being formed into an aggregate as a bond between the nucleic acid and the cationic polymer holds the bond graft, the bone grafting composition can serve as an ideal scaffold to maintain a bone regeneration space, like an extracellular matrix. In addition, when implanted to a lesion, the bone grafting composition can increase a cushioning force to counteract a physiological stress such as skeletal compression, tensile stress, torsion, and bending in vivo.

The bone grafting composition is formed into an aggregate, thus increasing the convenience of bone grafting. The nucleic acid and cationic polymer in the bone grafting composition are biocompatible and biodegradable.

The bone grafting is to replace missing bone due to various reasons such as trauma, infection, degenerative changes, tumorectomy, dental disease, etc., by filling a void volume within a skeletal structure and stimulating the formation of new bone and may find applications in the oral surgery or orthopedics fields in order to treat various bone defects or damage, including the following conditions: dental treatment or transplantation of teeth to restore the function of teeth; the occurrence of periodontal diseases such as periodontitis, incurable bone diseases, metabolic bone diseases, bone aplasia, complex bone fracture, or multiple bone fracture; fusion of bones and joints in the backbone; bone damage due to infection, senescence, and other physiological causes or various accidents; application to a void volume in a damaged bone; remedy for a bone around an insert such as an artificial joint; necessity for filling a void volume of the missing bone caused by bone disease including bone tumor and for synostosis and articular fixation; and the occurrence of deformity caused by trauma in articular cartilage, craniofacial skeleton, auricular cartilage, nasal bone, and joints, teratosis, removal of foreign matter, and senescence. The bone may be any human bone.

In addition, the present invention relates to a bone grafting kit for manufacturing the bone grafting composition, the kit comprising: a-1) a nucleic acid solution; b) a bone graft; and c-1) a cationic polymer solution.

The bone grafting kit may be stored in a form of a syringe or vial containing a-1) the nucleic acid solution, b) the bone graft, and c-1) the cationic polymer solution therein, or in a form capable of immediately injecting or implanting the bone grafting composition to a bone defect.

Alternatively, the bone grafting kit may be designed to contain a-1) the nucleic acid solution, b) the bone graft, and c-1) the cationic polymer solution in respective vials, separately.

In the bone grafting kit, a-1) the nucleic acid solution and b) the bone graft may be mixed in a vial while c-1) the cationic polymer solution may be contained in a separate vial, or a vial containing a mixture of c-1) the cationic polymer solution and b) the bone graft and a vial containing a-1) the nucleic acid are separately stored.

In addition, the bone grafting kit may be designed to contain a-2) a powder form of nucleic acid, b) a bone graft, c-2) a powder form of a cationic polymer, d) a buffer for dissolving the nucleic acid, and e) a buffer for dissolving the cationic polymer in respective vials, separately.

In the bone grafting kit, the nucleic acid and the cationic polymer may be separately stored as solutions in respective buffers or in their own crystalline forms, such as powders, but without limitations thereto.

The buffer for dissolving d) the nucleic acid may be distilled water, sodium chloride, sodium hydrogen phosphate, sodium dihydrogen phosphate dihydrate, sodium phosphate dibasic dodecahydrate, magnesium chloride, potassium chloride, phosphate buffer saline (PBS), HEPES (N-(2-hydroxyethyl)-piperazine-N'-2-ethanesulfonic acid), glycerol-3-phosphate, or a mixture thereof and particularly sodium chloride, sodium hydrogen phosphate, sodium dihydrogen phosphate dehydrate, or a mixture thereof, but is not limited thereto.

The buffer for dissolving e) the cationic polymer may be an acidic buffer and particularly, distilled water, acetic acid, hydrochloric acid, ascorbic acid, nitric acid, lactic acid, or a mixture thereof, and more particularly lactic acid, acetic acid, or a mixture thereof, but is not limited thereto.

The bone graft may be included, together with the other component, in the bone grafting kit and may be a commercially available one and thus purchased separately.

Concentrations of the nucleic acid solution may be determined according to the judgement of a person skilled in the art. Particularly, the nucleic acid solution may have a concentration of 0.5 % by weight or higher, but without limitations thereto. However, it is difficult to prepare a nucleic acid solution having a concentration of 2 % by weight because such an amount of the nucleic acid is unlikely to be dissolved.

Concentrations of the cationic polymer solution may be determined according to the judgement of a person skilled in the art. Particularly, the cationic polymer solution may have a concentration of 0.5 % by weight or higher. The cationic polymer may be dissolved to the maximum solubility.

The bone grafting kit may further comprise a bone morphogenesis activator, and the bone morphogenesis activator may be contained in a form of a solution or a powder and stored in a separate vial.

When including a powder form of the bone morphogenesis activator, the bone grafting kit may further comprise a buffer for dissolving the bone morphogenesis activator.

The buffer for dissolving the bone morphogenesis activator may be a buffer that allows the bone morphogenesis activator to retain the activity thereof. Particularly, the buffer may be a liquid containing a nutrient, such as phosphate buffer saline (PBS), physiological saline, a cell culture medium, a glucose solution, a branched-chain amino acid supplement, and Hartmann's solution and Hartmann's Dex used as Ringer's solutions, and may be used provided that it does not make an osmotic pressure after infusion into the body.

### Advantageous Effects

The present invention relates to a bone grafting composition comprising a nucleic acid, a bone graft, and a cationic polymer and a bone grafting kit for manufacturing the same. More particularly, the bone grafting composition is formed into an aggregate as the bone graft is held by a bond between the nucleic acid and the cationic polymer in the composition. Having an increased cushioning force, the aggregate was found to function as a scaffold and to stimulate the formation of new bone at grafted sites. In addition, the bone grafting composition of the present invention has increased cohesiveness and can be applied to bone grafting irrespective of shapes of grafted sites, thus improving the convenience of bone grafting.

Functioning as an effective scaffold for maintaining a bone regeneration space and growing cells to induce bone morphogenesis, the bone grafting composition of the present invention is expected to find advantageous applications to the treatment of various bone diseases.

### Brief Description of the Drawings

FIG. 1 shows whether an aggregate is formed from (A) a bone graft alone, (B) composition 1 comprising a mixture of a nucleic acid and a chitosan, (C) composition 2 prepared by mixing a nucleic acid with a chitosan and then with a bone graft, and (D) composition 3 prepared by mixing a nucleic acid with a bone graft and then with a chitosan.
FIG. 2 shows whether a bone graft is aggregated according to mixing ratios of a nucleic acid and a cationic polymer.
FIG. 3 shows whether a bone graft is aggregated according to concentrations and mixing ratios of a nucleic acid and a cationic polymer.
FIG. 4 shows cohesiveness and cushioning force of bone grafting compositions prepared from (A) a bone graft alone, without a nucleic acid and a cationic polymer, (B) composition 1 comprising a mixture of a nucleic acid and a chitosan, and (C) composition 3 prepared by mixing a nucleic acid with a bone graft and then with a chitosan.

### Mode for Carrying Out the Invention

Hereinafter, the present invention will be described in detail with reference to examples. These examples are only for illustrating the present invention more specifically, and it will be apparent to those skilled in the art that the scope of the present invention is not limited by these examples.

### <EXPERIMENTAL EXAMPLE 1: Aggregation of Bone Graft by Nucleic Acid and Cationic Polymer>

A polynucleotide (hereinafter, referred to as nucleic acid), commercially available from PharmaResearch Products, Co. Ltd., was used as a nucleic acid. In 9.8 ml of a buffer (0.8% sodium chloride, 0.25% sodium hydrogen phosphate, 0.028% sodium dihydrogen phosphate dihydrate), 0.2 g of the nucleic acid was dissolved at 40-70°C for 30-60 min using a heating stirrer to give a 2 % by weight nucleic acid solution.

Chitosan was used as a cationic polymer. In 9.8 ml of 0.12 M lactic acid, 0.2 g of chitosan was dissolved using a stirrer to give a 2 % by weight chitosan solution.

A demineralized xenogeneic bone derived from cattle was used as a bone graft.

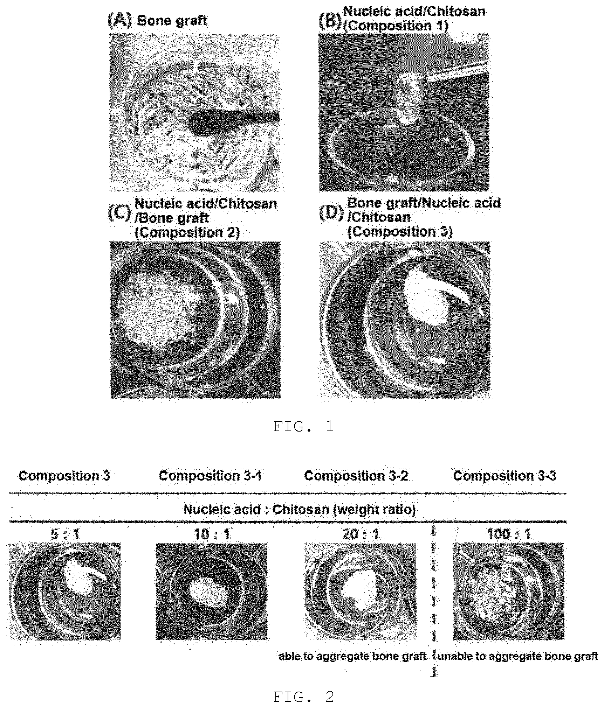

The nucleic acid solution, the chitosan solution, and the bone graft prepared above were mixed as shown in Table 1, below. The resulting compositions were analyzed for physical properties and for aggregation of the bone graft, and the results are depicted in FIG. 1.

**TABLE 1**

| Composition | Preparation |
|---|---|
| Composition 1 | Mix 1.25 ml of 2 % by weight nucleic acid solution with 0.25 ml of 2 % by weight of chitosan solution |
| Composition 2 | Mix 1.25 ml of 2 % by weight nucleic acid solution with 0.25 ml of 2 % by weight of chitosan solution and then with 0.5 g of bone graft |
| Composition 3 | Mix 0.5 g of bone graft with 1.25 ml of 2 % by weight nucleic acid solution and then with 0.25 ml of 2 % by weight chitosan solution |

As shown in FIG. 1, composition 1, which was prepared by mixing the nucleic acid solution and the chitosan solution (FIG. 1B), might appear to be a gel, but was not a gel and showed a physical property such that it could be easily handled with tweezers. In composition 2 (FIG. 1C), which was prepared by mixing the nucleic acid solution with the chitosan solution and then with the bone graft, the bone graft was not aggregated by the nucleic acid solution and chitosan solution mixture and was observed to be dispersed, like the bone graft not mixed with any material. In contrast, composition 3 (FIG. 1D), which was prepared by mixing the bone graft with the nucleic acid solution and then with the chitosan solution was observed to aggregate the bone graft.

Although not shown in FIG. 1, a composition prepared by mixing the bone graft with the chitosan solution and then with the nucleic acid solution was also observed to aggregate the bone graft, as in composition 3.

Even when chitin, polyethylene amine, poly-L-lysine, and polyallylamine were used as cationic polymers, the same results were obtained although not stated in detail.

Taken together, the data obtained in this Example indicated that the bond between the nucleic acid and the cationic polymer induces the aggregation of the bone graft material and the order of mixing the nucleic acid, the cationic polymer, and the bone graft material is also important for the aggregation of the bone graft aggregation.

### <EXPERIMENTAL EXAMPLE 2: Aggregation of Bone Graft According to Mixing Ratio of Nucleic Acid and Cationic Polymer>

An examination was made to see whether or not the nucleic acid and chitosan mixture-induced bone graft aggregation confirmed in Experimental Example 1 was influenced by mixing ratios of nucleic acid and chitosan. To this end, the bone graft was analyzed for aggregation in the presence of nucleic acid and chitosan mixed at various ratios. In this regard, the concentration of chitosan was changed while the concentration of nucleic acid was fixed.

A 2 % by weight nucleic acid solution and 2 % by weight, 1 % by weight, 0.5 % by weight, and 0.1 % by weight chitosan solutions were prepared in the same manner as in Experimental Example 1. Compositions containing nucleic acid and chitosan at the concentrations indicated in Table 2, below, were made by mixing 0.5 g of bone graft with 1.25 ml of a 2 % by weight nucleic acid solution, followed by adding 0.25 ml of each of the chitosan solutions having the concentrations while aggregation of the bone graft was monitored. The results are depicted in FIG. 2.

**TABLE 2**

| Composition | Final Conc. in Composition (wt%) | | Mixing ratio (weight ratio) | |
|---|---|---|---|---|
| | Nucleic acid | Chitosan | Nucleic acid | Chitosan |
| Composition 3 | 1.667 | 0.333 | 5 | 1 |
| Composition 3-1 | 1.667 | 0.167 | 10 | 1 |
| Composition 3-2 | 1.667 | 0.083 | 20 | 1 |
| Composition 3-3 | 1.667 | 0.017 | 100 | 1 |

As shown in FIG. 2, aggregation of the bone graft was found to be dependent on mixing ratios between nucleic acid and chitosan. That is, when nucleic acid and chitosan in bone grafting compositions were mixed at weight ratios of 5:1 (composition 3), 10:1 (composition 3-1), and 20:1 (composition 3-2), the bone graft aggregated. In contrast, when nucleic acid and chitosan was mixed at 100:1 (composition 3-3), the bone graft did not aggregate, but was dispersed.

The data obtained in this experiment indicate that mixing ratios between nucleic acids and cationic polymers have important influences on the aggregation of the bone graft.

### <EXPERIMENTAL EXAMPLE 3: Aggregation of Bone Graft According to Concentration of Nucleic Acid and Cationic Polymer>

Through Experimental Example 2, it was observed that mixing ratios between nucleic acid and chitosan are important for the aggregation of bone grafts. In addition, an experiment was made to confirm the aggregation of bone grafts according to concentrations of nucleic acids and chitosan.

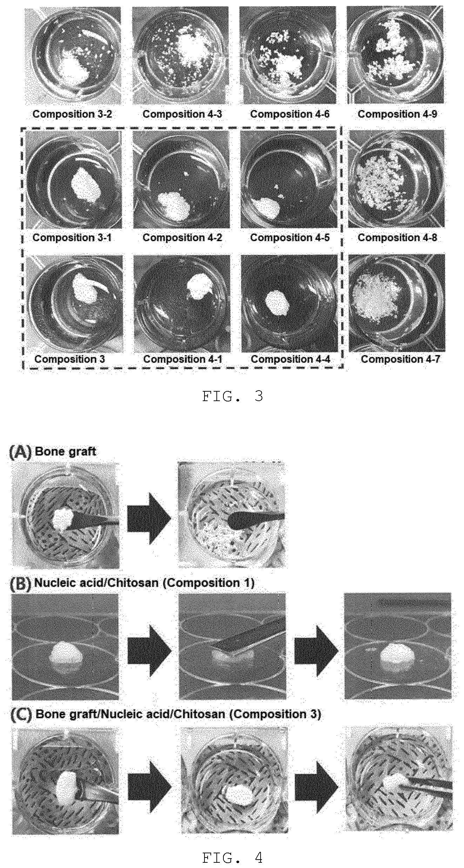

Chitosan solutions having 2 % by weight, 1 % by weight, and 0.5 % by weight concentrations and nucleic acid solutions having 2 % by weight, 1.5 % by weight, 1 % by weight, and 0.5 % by weight concentrations were prepared in the same manner as in Experimental Example 1 for the nucleic acid and chitosan solutions. Compositions containing nucleic acid and chitosan at the concentrations indicated in Table 3, below, were made by mixing 0.5 g of bone graft with 1.25 ml of each of the nucleic acid solutions having the concentrations, followed by adding 0.25 ml of each of the chitosan solutions having the concentrations while aggregation of the bone graft was monitored. The results are depicted in FIG. 3.

**TABLE 3**

| Composition | Final Conc. in Composition (wt%) | | Mix Ratio weight ratio) | |
|---|---|---|---|---|
| | nucleic acid | chitosan | nucleic acid | chitosan |
| Composition 3 | 1.667 | 0.333 | 5 | 1 |
| Composition 3-1 | 1.667 | 0.167 | 10 | 1 |
| Composition 3-2 | 1.667 | 0.083 | 20 | 1 |
| Composition 4-1 | 1.250 | 0.333 | 3.6 | 1 |
| Composition 4-2 | 1.250 | 0.167 | 7.2 | 1 |
| Composition 4-3 | 1.250 | 0.083 | 15 | 1 |
| Composition 4-4 | 0.830 | 0.333 | 2.4 | 1 |
| Composition 4-5 | 0.830 | 0.167 | 4.7 | 1 |
| Composition 4-6 | 0.830 | 0.083 | 10 | 1 |
| Composition 4-7 | 0.420 | 0.333 | 1.2 | 1 |
| Composition 4-8 | 0.420 | 0.167 | 2.3 | 1 |
| Composition 4-9 | 0.420 | 0.083 | 5 | 1 |

Compositions 3, 4-5, and 4-9 were different in concentrations of nucleic acid and chitosan, but similar in mixing ratios therebetween. As shown in FIG. 3, the bone graft was not aggregated, but dispersed in composition 4-9 whereas the bone graft was aggregated in compositions 4-5 and 3. These results were true of compositions 4-4 and 4-8 and compositions 3-1 and 4-6.

In this experiment, it was found that the aggregation of the bone graft was dependent on the final concentrations of nucleic acid and cationic polymer as well as the mixing ratios of nucleic acid and cationic polymer.

The data obtained in Experimental Examples 1 to 3 show that the bone grafting composition of the present invention preferably contains a nucleic acid in an amount of 0.001-2 % by weight and a cationic polymer in an amount of 0.001-2 % by weight, based on the total weight thereof, with the nucleic acid and the cationic polymer mixed at a ratio of 1-20:1 (weight ratio).

### <EXPERIMENTAL EXAMPLE 4: Effect of Nucleic Acid and Cationic Polymer on Cohesiveness of Bone Graft and Buffering Powder of Bone Grafting Composition>

An examination was made of effects of the nucleic acid and cationic polymer on the cohesiveness of the bone graft and the cushioning force of the bone grafting composition. In this regard, compositions 1 and 3 of Experimental Example 1 were employed and monitored for morphological change upon application of a force thereto. The results are depicted in FIG. 4.

As can be seen in FIG. 4, the bone graft, when existing alone (FIG. 4A), did not aggregate and was easily dispersed with the application of even a small force thereto. Composition 1 which contained a mixture of nucleic acid and chitosan, but not bone graft (FIG. 4B), was not dispersed even in the presence of a force applied thereto and was observed to have a restoring force to return back to the original shape and a cushioning force upon removal of the applied force. For composition 3 containing a mixture of nucleic acid, chitosan, and bone graft (FIG. 4C), the bone graft in the mixture was aggregated in contrast to the bone graft alone and retained the morphology thereof without being dispersed even when a force was applied thereto. Thus, the composition was observed to have a cohesive force and a cushioning force.

In this experiment, it was thus found that the bone grafting composition comprising a nucleic acid, a cationic polymer, and a bone graft according to the present invention has a cohesive force to aggregate the bone graft which can be thus prevented from being dispersed by an external force, and a cushioning force to counteract a physical impact in the course of bone grafting and bone regeneration, whereby the bone graft can retain a necessary shape and improve the convenience of bone grafting.

### <EXPERIMENTAL EXAMPLE 5: Maintenance of Cohesiveness of Bone Grafting Composition over Time>

The bone grafting composition of the present invention was monitored for a decrease in cohesiveness with time. Changes in the total weight of the composition were used as criteria for the maintenance of cohesiveness because the bone graft is dispersed as the cohesiveness of the bone grafting composition is decreased.

In this experiment, composition 3 of Experimental Example 1 was employed. Just after being prepared, composition 3 was measured for total weight every 30 minutes. The results are given in Table 4, below.

**TABLE 4**

| | | | | | | |
|---|---|---|---|---|---|---|
| Time (min) | 0 | 30 | 60 | 90 | 120 | 150 |
| Weight (g) | 0.7 | 0.69 | 0.7 | 0.69 | 0.68 | 0.69 |

As can be understood from the data of Table 4, the total weight of the bone grafting composition according to the present invention did not undergo a large change with time. Thus, the cohesiveness of the bone grafting composition according to the present invention lasted for a long period of time.

Accordingly, the bone grafting composition of the present invention guarantees high stability without a morphological change during a grafting procedure for a long time, and allows the bone graft to last in an aggregate form without being broken for a long period of time, thereby making the bone grafting procedure convenient.

### <EXPERIMENTAL EXAMPLE 6: Induction of Bone Morphogenesis>

In order to examine the effect of the bone grafting composition of the present invention on bone morphogenesis, the bone grafting composition was implanted to a site free of bone growth and observed for the formation of new bone.

In this regard, compositions 2 and 3 of Example 1 were implanted to bone growth-free abdominal muscles in rats, followed by suturing the muscles and skins. As a control, the bone graft was used alone. After the rats were bred, the entire implanted abdominal muscle area was excised, demineralized, and embedded into paraffin to construct tissue specimens. The tissue specimens were subjected to Giemsa staining and H&E staining and quantitatively analyzed for new bone tissue to determine the formation of new bone.

In the control treated with the bone graft alone, no new bones were found. For composition 2, new bones were almost not found, like the control. In contrast, composition 3 was found to significantly increase the amount of new bones, unlike the control and composition 2.

Taken together, the data obtained in this experiment indicate that the bone grafting composition of the present invention has the effect of inducing bone morphogenesis.

### <EXAMPLE 1: Construction of Bone Grafting Kit>

### Example 1-1: Preparation of nucleic acid solution

In 9.8 ml of a buffer (0.8% sodium chloride, 0.25% sodium hydrogen phosphate, 0.028% sodium dihydrogen phosphate dihydrate), 0.2 g of a nucleic acid was dissolved at 40-70°C for 30 to 60 minutes using a heating stirrer to prepare a 2 % by weight nucleic acid solution.

### Example 1-2: Preparation of cationic polymer

As a cationic polymer, chitosan was used.

In 9.8 ml of a 0.12 M lactic acid solution, 0.2 g of chitosan was dissolved using a stirrer to prepare a 2 % by weight chitosan solution.

### Example 1-3: Construction of bone grafting kit

A bone grafting kit according to the present invention was constructed by combining a vial storing the nucleic acid solution prepared in Example 1-1 and a vial storing the chitosan (cationic polymer) solution prepared in Example 1-2. In this regard, a vial storing a bone graft may be included in the kit.

## Claims

1. A bone grafting composition, comprising:
a) a nucleic acid;
b) a bone graft; and
c) a cationic polymer,
wherein a) the nucleic acid and c) the cationic polymer form a bond which holds b) the bone graft to form an aggregate, said bone grafting composition being prepared by:
a process comprising
a first step of mixing a) the nucleic acid with b) the bone graft to give a nucleic acid-bone graft mixture and
a second step of adding c) the cationic polymer to the nucleic acid-bone graft mixture to hold the bone graft to form an aggregate; or
a process comprising
a first step of mixing c) the cationic polymer with b) the bone graft to give a cationic polymer-bone graft mixture and
a second step of adding a) the nucleic acid to the cationic polymer-bone graft mixture to hold the bone graft to form an aggregate.

2. The bone grafting composition of claim 1, wherein the bond is an ionic bond.

3. The bone grafting composition of claim 1, wherein the nucleic acid is mixed at a weight ratio of 1-20:1 with the cationic polymer.

4. The bone grafting composition of claim 1, wherein the nucleic acid is contained in an amount of 0.001-2 % by weight, based on the total weight of the bone grafting composition.

5. The bone grafting composition of claim 1, wherein the nucleic acid is a deoxyribonucleic acid (DNA), a ribonucleic acid (RNA), or a mixture thereof.

6. The bone grafting composition of claim 1, wherein the nucleic acid has a molecular weight of 1-100,000 kDa.

7. The bone grafting composition of claim 1, wherein the cationic polymer is contained in an amount of 0.001-2 % by weight, based on the total weight of the bone grafting composition.

8. The bone grafting composition of claim 1, wherein the cationic polymer is at least one selected from the group consisting of chitosan, polyethylene amine, poly-L-lysine, and polyallylamine.

9. The bone grafting composition of claim 1, wherein the cationic polymer is chitosan.

10. The bone grafting composition of claim 1, wherein the bone graft is an autogeneic demineralized bone matrix, an allogeneic demineralized bone matrix, a xenogeneic demineralized bone matrix, a synthetic bone graft, an autologous bone homogenate, or a mixture thereof.

11. The bone grafting composition of claim 1, further comprising a bone morphogenesis activator.

12. The bone grafting composition of claim 11, wherein the bone morphogenesis activator is a growth factor, a hemostatic, or a mixture thereof.

13. The bone grafting composition of claim 12, wherein the growth factor is at least one selected from the group consisting of bone morphogenetic protein (BMP), bone sialoprotein, transforming growth factor (TGF), platelet-derived growth factor (PDGF), platelet rich plasma (PRP), fibroblast growth factor (FGF), dentin sialoprotein, polydeoxyribonucleotide, heparin-binding EGF-like growth factor (HB-EGF), cadherin EGF LAG seven-pass G-type receptor 3, and osteoblast specific cadherin (OB-cadherin) .

14. The bone grafting composition of claim 12, wherein the hemostatic is at least one selected from the group consisting of thrombin, thromboplastin, fibrinogen, casein-kinase II, tissue factor, epinephrine, gelatin, vitamin K, calcium chloride, aluminum chloride-hexahydrate, and aluminum sulfate.

15. A bone grafting kit for preparing the bone grafting composition of claim 1, the bone grafting kit comprising:
a-1) a nucleic acid solution;
b) a bone graft; and
c-1) a cationic polymer solution.

16. The bone grafting kit of claim 15, further comprising a bone morphogenesis activator.
